# EUROPEAN PATENT APPLICATION

(11) **EP 2 465 517 A1**
(43) Date of publication of application: **20.06.2012**
(21) Application number: 09784137.3
(22) Date of filing: 12.08.2009
(51) Int. Cl.: A61K 36/63, A61P 3/06

(54) **COMPOSITION BASED ON EXTRA VIRGIN OLIVE OILS**

(71) Applicant: Bioaveda, S.L., 23005 Jaén (ES)
(72) Inventor: GARCÍA VILLARRUBIA, Vicente, E-23005 Jaén (ES); PEREZ BAÑASCO, Vicente, E-23005 Jaén (ES); TORRES MORALES, Juan, E-23005 Jaén (ES); GIL CUNQUERO, José Manuel, E-23005 Jaén (ES); BORREGO UTIEL, Francisco, E-23005 Jaén (ES); LLACER GALLACH, José Miguel, E-23005 Jaén (ES); ALBERTO COSTA, Luís, E-23005 Jaén (ES); PINO PINO, Mª Dolores, E-23005 Jaén (ES); VIDAL ASENSI, Santiago, E-23005 Jaén (ES); CISTERNA CÁNCER, Ramón, E-23005 Jaén (ES); CABEZAS SEBASTIÁN, Juan Jesús, E-23005 Jaén (ES); HERNÁNDEZ MARAVER, José A., E-23005 Jaén (ES)
(74) Representative: Pons Ariño, Angel
(86) International application number: PCT/ES2009/070338
(87) International publication number: WO 2011/018534

(57) **Abstract**

The invention relates to a composition based on a mixture of different olive oils, preferably ecologically cultivated virgin and extra virgin olive oils, and to the use thereof for treating or preventing different disorders in humans and animals. In addition, the invention relates to a dermatologic/dermocosmetic preparation comprising the composition of the invention and to the use thereof for the treatment of skin disorders in humans and animals. In these systemic and/or skin disorders underlying metabolic and immuno-inflammatory-oxidative alterations exist that can be positively modified through the systemic use (oral) or topical use (cutaneous) of the composition of the present invention. The special physical-chemical structure of the composition allows for improved digestive and cutaneous conditions.

## Description

This invention relates to a composition based on a mixture of extra virgin olive oils of different varieties and to the use thereof in the prevention and/or treatment of different conditions in humans and animals.

### PRIOR STATE OF THE ART

In Spain, there are many varieties and sub-varieties of olive trees/olives, which leads to the existence of different varieties and sub-varieties of olive oils (OO) in their two most natural presentations, virgin and extra virgin. Not only do these variations affect the secondary components (polyphenols, squalene and vitamin E, amongst them), but the fatty acids (FAs), which are primary components of OO, differ from one variety to the other, affecting all olive trees. These fluctuations in the FA contents (oleic, linoleic, palmitic, stearic, etc.) are also observed in other non-Spanish varieties, and are even detected between olive trees from the same farm.

The chemical and organoleptic quality of OO is influenced not only by the genetic or epigenetic factors inherent in the olive tree varieties, but also by agrological and climactic factors, as well as by those related to the extraction methodology. These factors include:
- Agronomic factors, such as altitude or soil salinity
- The temperature and the water fallen during the harvest
- The degree of maturity of the olives at the time of harvesting
- The history of the olives from the time of harvesting
- The extraction methodology: the higher the heat, the higher the yield (greater quantity of oil), but also the fewer the healthy properties.

As a significant example of the influence of these factors, cf. the extreme variations in polyphenol contents between two varieties of OO used in two clinical trials (Ramírez-Tortosa MC, et al. Extra-virgin olive oil increases the resistance of LDL to oxidative more than refined olive oil in free-living men with peripheral vascular disease. J Nutr 1999; 129: 2177-83*.* Fito M, et al. Antioxidant effect of virgin olive oil in patients with stable coronary heart disease: a randomised, crossover, controlled clinical trial. Atherosclerosis 2005; 181: 149-58).

OO has traditionally been used as a food, and recently its utility as a functional food has begun to be considered, primarily associated with the so-called Mediterranean Diet (MD). On the basis of epidemiological arguments, the conclusion was reached that this type of diet increased the quality of life, reduced the incidence of morbid diseases and increased overall survival in consumers (Willett WC, et al. Mediterranean diet pyramid: a cultural model for healthy eating. Am J Clin Nutr 1995; 61 (suppl 6): 1402S-6S)*.* The healthy effects of this form of nutrition are partly due to OO, in particular to some of its components, such as monounsaturated (MUFA) or polyunsaturated fatty acids (PUFA), and compounds with antioxidant properties, such as polyphenols, the consumption whereof is associated with a lower risk of suffering from cardiovascular diseases (Lapointe A, et al. Effects of dietary factors on oxidation of low-density lipoprotein particles. J Nutr Biochem 2006; 17: 645-58*).*

The predominant MUFA in OO is oleic acid, although the proportions of this component in different OOs vary as a function of the factors described above. There are many studies on the effects of oleic acid from OO and other vegetable oils such as sunflower oil, on cholesterol levels (total cholesterol or TC, low-density lipoprotein associated cholesterol or LDL-c, and high-density protein associated cholesterol or HDL-c), wherein reductions in the levels of TC and LDL-c are observed, but the levels of HDL-c hardly suffer any variations (Gardner CD, Kraemer HC. Monounsaturated versus polyunsaturated dietary fat and serum lipids. Artherioscler Thromb Vasc Biol 1995; 15: 1917-27*.* Ródenas S, et al. Dietary exchange of an olive oil and sunflower oil blend for extra virgin olive oil decreases the estimate cardiovascular risk and LDL and apolipoprotein All concentrations in postmenopausal women. J Am Coll Nutr 2005; 24: 361-9*).* It is worth noting that the effect of OO in reducing the levels of TC and LDL-c is more clearly seen in healthy subjects than in subjects with pathologies, such as chronic kidney disease (CKD), hypertension, diabetes or hyperlipidaemia.

Other studies focused on other pathologies, such as hypertension or insulin resistance, show variable results with respect to OO intake. Whereas some studies show that prolonged, continuous OO intake reduces the risk of developing hypertension (Alonso A, et al. Olive oil consumption and reduced incidence of hypertension: the SUN study. Lipids 2004; 39: 1233-8*),* OO does not reduce blood pressure in diabetic patients or patients with CKD (Svensson M, et al. The effect of n-3 fatty acids on plasma lipids and lipoproteins and blood pressure in patients with CRF. Am J Kidney Dis 2004; 44: 77-83).

Insulin resistance (IR), glucose intolerance and hyperinsulinaemia are, jointly with obesity and hypertension, the main characteristics of the so-called metabolic syndrome, which usually precedes the establishment of diabetes mellitus (DM) or type 2 diabetes (DM-2), with the consequent risk of morbidity and mortality due to cardiovascular disease (CVD). In general, it is believed that IR increases with the consumption of saturated fatty acids and decreases with n-3 PUFA, whereas the effects of n-6 PUFA (present in OO) are controversial.

Most studies on the effects of OO on animals suffering from metabolic syndrome do not show positive effects of the OO intake (Mori Y, et al. Influence of highly purified eicosapentaenoic acid ethyl ester on insulin resistance in the Otsuka Long-Evans Tokushima Fatty rat, a model of spontaneous non-insulin-dependent diabetes mellitus. Metabolism 1997; 46: 1458-64)*,* and some even conclude that 00-rich diets exacerbate obesity and may cause certain pathologies (Buettner R, et al. Defining high-fat-diet rat models: metabolic and molecular effects of different fat types. J Mol Endocrinol 2006; 36: 485-501)*.*

Taking into consideration the variability in, and in some cases contradictions between, the results of the above-mentioned studies, it may be concluded that, despite maintaining a common basic structure of primary components (fatty acids) and secondary components (polyphenols, vitamin E and others), the different OOs exhibit significant quantitative, and sometimes qualitative, variations in their different constituents, and that said variations may be associated with both genetic and agronomic conditions, such as the processes that lead to the production of OO: farming techniques, harvesting and storage stages, as well as grinding, beating, preservation, etc. methods.

These differences in the composition of OO are translated into variations in the biological effects on its consumers. In fact, OO does not cause beneficial effects, in regards to the clinical parameters described above, in those patients with the highest risk of cardiovascular disease; i.e. in patients with CKD (Svensson M, et al. The effect of n-3 fatty acids on plasma lipids and lipoproteins and blood pressure in patients with CRF. Am J Kidney Dis 2004; 44: 77-83*.* Svensson M, et al. The effect of n-3 fatty acids on lipids and lipoproteins in patients treated with chronic hemodialysis: a randomized placebo-controlled intervention study. Nephrol Dial Transplant 2008 [23: 2918-24*].* Vernaglione L, et al. Omega-3 polyunsaturated fatty acids and proxies of cardiovascular disease in hemodialysis: a prospective cohort study. J Nephrol 2008; 21: 99-105)*.*

Another, less known aspect, which is furthermore controversial, relates to the immunological alterations caused by OO. Although the antioxidant and anti-inflammatory actions seem to be well-defined, there are no *in vivo* data from humans defining any immunological activity of OO which might be responsible for the plethora of clinical effects described.

Finally, the least known aspect of OO is its topical use. Although olive oil has been traditionally considered for external use as a protective, moisturising agent, there are hardly any basic scientific or clinical arguments that justify these applications. However, the increased knowledge about the molecular structure of the skin, with its wealth of lipids and its complicated cell renewal and differentiation metabolic processes, together with the improvement in OO preparation methods, currently make it possible to put the pieces of the picture together, rationalizing certain assumptions about OO and the skin present in so-called "folk medicine". (Villarrubia VG, et al. Barrera epidérmica y nutrición. I. La conexión PPAR y enzimopatología. Personalizando la dermatitis atópica*. Villarrubia VG, et al. Barrera epidermica y nutrición lipídica. II. La conexión PPAR e Inmunopatología inflamatoria. EI papel de la proteína epidérmica fijadora de ácidos grasos).*

It would therefore be necessary to harmonise one or several OOs on the basis of rational mixtures (Magistral Formulations or "coupages") of olive oils of different varieties (genetic origin) and/or harvests, designed to provide a chemical composition that ensures a certain biological function; i.e. an optimised OO mixture that allows for the reproducibility of its biological actions in humans, with the consequent therapeutic repercussions in models of high pathological commitment.

### DESCRIPTION OF THE INVENTION

This invention provides a composition based on a mixture of different types of olive oil, preferably from ecological farms and subject to strict quality controls, and to the use thereof in the prevention or treatment of various disorders in humans and animals.

A first aspect of this invention relates to a composition that comprises, at least, the following varieties of extra virgin olive oil:
■ Extra virgin olive oil of the Picual variety in a proportion of between 42% and 62% by volume
■ Extra virgin olive oil of the Arbequino variety in a proportion of between 20% and 40% by volume
■ Extra virgin olive oil of the Cornicabra variety in a proportion of between 8% and 28% by volume
■ Extra virgin olive oil of the Hojiblanca variety in a proportion of between 0% and 5% by volume
■ Extra virgin olive oil of the Empeltre variety in a proportion of between 0% and 5% by volume.

Preferably, the olive oils in the composition described above are obtained from ecological agriculture.

In order to obtain the compositions of this invention, it is necessary to begin by applying strict Traceability Standards in the Fields and Mills, which makes it possible to ensure the quality of the OOs designed to prepare them; selecting farms where the use of environment-friendly ecological methods have is guaranteed ensures oil purity and the absence of potentially toxic substances from the olive oils used to obtain the compositions of this invention.

Another aspect of this invention relates to a composition such as that described to be used as a drug.

Another aspect of this invention relates to a pharmaceutical preparation that comprises the composition described above and a pharmaceutically acceptable adjuvant or carrier.

The "pharmaceutically acceptable adjuvants" or "carriers" that may be used in said compositions are the carriers known to those skilled in the art.

Examples of pharmaceutical preparations include any solid composition (tablets, pills, capsules, granules, etc.) or liquid composition (solutions, suspensions or emulsions) for oral, topical or parenteral administration, preferably oral or topical administration, or both simultaneously. The pharmaceutical compositions that contain the compounds of this invention may also be formulated in the form of liposomes or nanospheres, controlled-release formulations or any other conventional release system.

Another aspect of this invention relates to the use of the composition described above for the manufacturing of a drug designed for the treatment or prevention of a disorder selected from a set of disorders associated with serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders caused by vascular stents or dialysis procedures, inflammatory or oxidative disorders, or immunological disorders.

The correct dosage of the composition will vary depending on several factors, such as the type of formulation used, the mode of application, the disorder to be treated, etc. Other factors must also be taken into consideration, such as age, body weight, sex, diet, time of administration, excretion speed, health condition of host, sensitivity in terms of reactions and severity of the disorder. Administration may be performed continuously or periodically within the maximum tolerated dose.

Preferably, the composition of this invention is used for the treatment or prevention of a metabolic or immunological/inflammatory/oxidative, degenerative or nutritional disorder, selected from chronic kidney disease (under dialysis or not), diabetes and insulin resistance, hypercholesterolaemia, dyslipidaemia, hypertension, hypoalbuminaemia and hypoproteinaemia, nutritional deficiencies (kwashiorkor, cachexia, anorexia and bulimia), co-adjuvant with treatment with cholesterol-reducing therapies, constipation, age-associated inflammatory processes, physical condition deficiencies (performance), dermatitis and dermatosis in all their forms, including hand eczema and bullous epidermolysis, vitiligo or disorders of the skin appendages (nails and hair).

Another aspect of this invention relates to the use of the composition described above as a co-adjuvant with different therapies. Preferably, said therapy is selected from the set of surgery, chemotherapy, radiotherapy, nutritional therapies, therapies with cholesterol-reducing agents, dialysis procedures and vascular stents, or administration of vaccines.

Another aspect of this invention relates to the use of the composition described above as a vehicle/carrier of drugs and/or nutraceutic agents and/or nutritional therapies.

The compositions of this invention may be used jointly with other active principles or therapies as combination therapy. The other active principles may be a part of the same composition or may be provided by a different composition, administered at the same time or at a different time.

Another aspect of this invention relates to the use of a composition such as that described above for the treatment or prevention of a disorder selected from a set of disorders associated to serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders caused by vascular stents or by dialysis procedures, inflammatory or oxidative disorders, or immunological disorders.

Another aspect of this invention relates to the use of the composition described above for the manufacturing of a nutraceutic agent or functional food.

In this invention, "nutraceutic agent" or "functional food" is understood to mean a food that has a beneficial effect on health. Likewise, the term nutraceutic agent may be applied to extracts or chemical compounds obtained from common foods. Examples of foods whereto nutraceutic properties have been attributed are olive oil, red wine, broccoli, soy, etc. Nutraceutic agents are normally used in nutritional mixtures and in the pharmaceutical industry. Just as some foods may be classified as nutraceutic, some nutritional supplements are also classified as such; for example, fatty acids such as omega-3 fatty acids derived from fish oil and some vegetable oils, or antioxidants and vitamins.

Another aspect of this invention relates to a method designed for the treatment or prevention of disorders in a mammal, preferably a human being, which comprises the administration of a therapeutically effective quantity of a composition such as that described above. Preferably, administration of the composition is performed by oral or topical route or both simultaneously.

In the sense used in this description, the term "therapeutically effective quantity" refers to the quantity of the composition of this invention calculated to produce the desired effect and, in general, will be determined, amongst other causes, by the characteristics of the composition, the patient's age, condition and history, the severity of the alteration or disorder, and the administration route and frequency.

Preferably, in said treatment method, the disorder is selected from a set of disorders associated with serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders caused by vascular stents or by dialysis procedures, inflammatory or oxidative disorders, or immunological disorders. More preferably, the metabolic or immunological/inflammatory/oxidative, degenerative or nutritional disorder is selected from chronic kidney disease (under dialysis or not), diabetes and insulin resistance, hypercholesterolaemia, dyslipidaemia, hypertension, hypoalbuminaemia and hypoproteinaemia, nutritional deficiencies (kwashiorkor, cachexia, anorexia and bulimia), co-adjuvant with treatment with cholesterol-reducing therapies, constipation, age-associated inflammatory processes, physical condition deficiencies (performance), dermatitis and dermatosis in all their forms, including hand eczema and bullous epidermolysis, vitiligo or disorders of the skin appendages (nails and hair).

Another aspect of this invention relates to a dermatological or dermocosmetic preparation that comprises at least between 1% and 95%, preferably between 5% and 90%, and more preferably between 10% and 80%, of the oil composition described above.

In a preferred embodiment, the dermatological/dermocosmetic preparation described above additionally comprises another ingredient selected from urea, shea butter, vitamin E, vitamin A, retinoic acid and the derivatives thereof, rose hip, aloe vera, bisabolol, lanolin or olive pit and/or seed and/or pulp extract or extract from the leaves or the pulp or other parts of the olive tree, as well as other active principles and/or extracts from plants and/or animals wherein the oil composition may act as a carrier/vehicle. Preferably, the other ingredient is in a proportion of between 0.05% and 95% by volume, more preferably of between 5% and 90% by volume and, even more preferably, of between 10% and 80% by volume.

The cosmetic or pharmaceutical preparations containing the composition of this invention may be used in different types of formulations for the topical or transdermal application thereof, such as, for example, without they being limited thereto, creams, oil and/or silicone emulsions in water, emulsions of water in oil and/or silicone, emulsions of the water/oil or silicone/water type, emulsions of the oil or silicone/water/oil or silicone type, oils, milks, balsams, foams, lotions, hydroalcoholic solutions, gels, liniments, sera, soaps, ointments, mousses, powders, sticks, pencils or vapourisers or aerosols (sprays), including leave-on and rinse-off formulations, and may also be incorporated, by means of techniques known to those skilled in the art, into different types of solid accessories, such as, for example, without they being limited thereto, wipes, hydrogels, adhesive (or non-adhesive) patches or face packs, or may be incorporated into different make-up products, such as, for example, without they being limited thereto, foundations, make-up remover lotions, make-up remover milks or eye bag correctors, amongst others.

Another aspect of this invention relates to a method designed for the treatment or prevention of a skin disorder in a mammal, preferably humans, dogs, cats and horses, which comprises topically applying a therapeutically effective quantity of the dermatological/dermocosmetic composition described above on the area to be treated. Preferably, the skin disorder is selected from atopic dermatitis, hand eczema, psoriasis, hyperkeratosis, pemphigus, vitiligo and other pigmentary disorders, processes that affect the skin and nails, bullous epidermolysis, burns caused by external agents, intrinsic and extrinsic skin ageing, iatrogenic disorders caused by vascular stents or by treatments with corticoids, cytostatic agents, retinoic acid and the derivatives thereof, psoralens associated with ultraviolet radiation (PUVA or PUVB) and the derivatives thereof, or other natural and/or synthetic substances for topical use, tumours or natural or iatrogenic infections of the skin and skin appendages.

Another aspect of this invention relates to the use of a dermatological/dermocosmetic preparation such as that described above for the treatment or prevention of a skin disease.

Another aspect of this invention relates to the use of a dermatological/dermocosmetic preparation such as that described above as a co-adjuvant in a therapy. Preferably, said therapy is selected from light therapies with laser or ultraviolet radiations or photochemotherapy, surgery, chemotherapy or radiotherapy, nutritional therapies, treatments with corticoids and other natural and/or synthetic substances for topical use.

The frequency of application of the dermocosmetic or pharmaceutical preparation that comprises the composition of the invention may vary widely, depending on each subject's needs; the application range suggested is between once a month and 10 times a day, preferably between once a week and 4 times a day, more preferably between three times a week and three times a day, even more preferably once or twice a day.

Throughout the description and the claims, the word "comprises" and the variants thereof are not intended to exclude other technical characteristics, additives, components or steps. For those skilled in the art, other objects, advantages and characteristics of the invention will arise partly from the description and partly from the practise of the invention. The following examples and drawings are provided for illustrative purposes, and are not intended to limit this invention.

### DESCRIPTION OF FIGURES

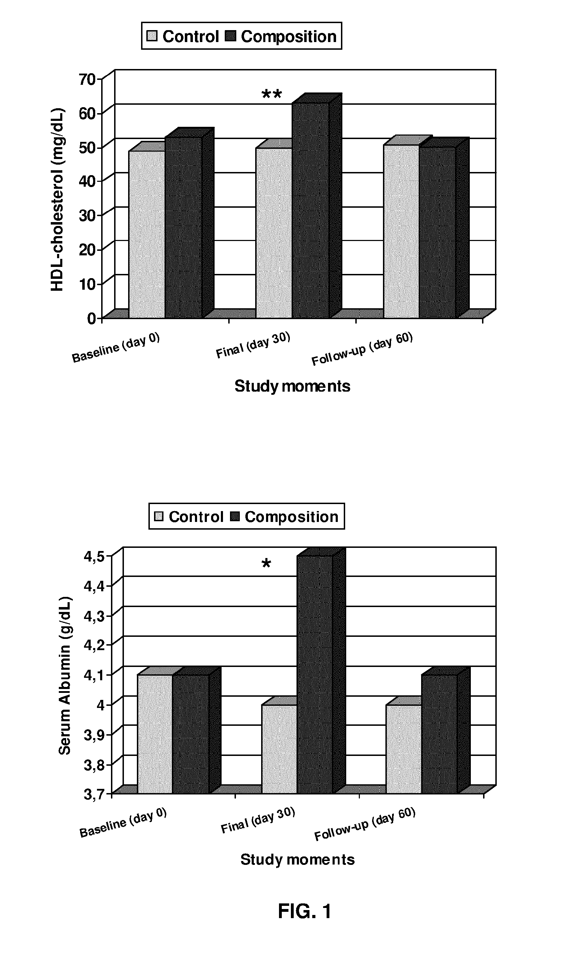

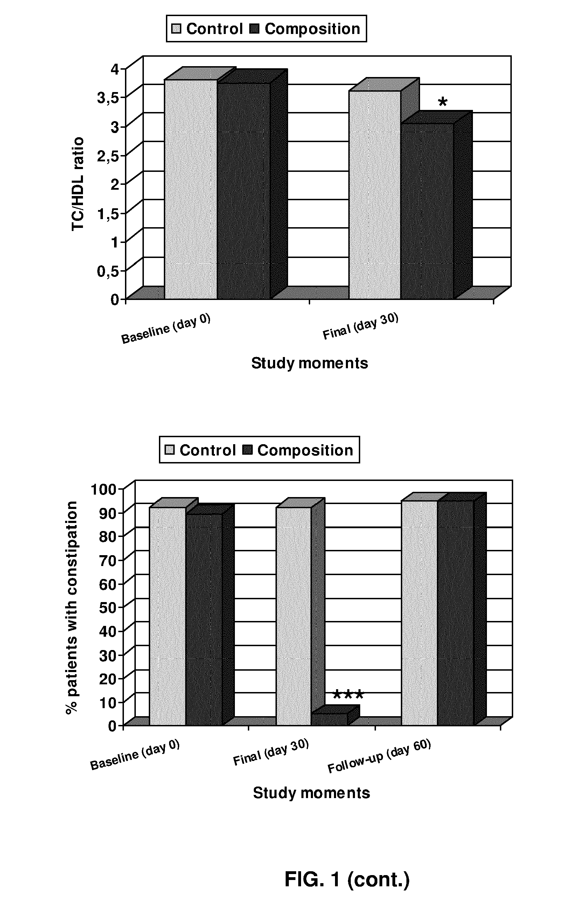

**Fig. 1****.** Evolutive changes of albumin and HDL-cholesterol serum levels, as well as of the total cholesterol/HDL-cholesterol ratio and constipation, seen in patients with Chronic Kidney Disease under treatment with a conventional olive oil (control group) or with the Composition. Both products were orally given at daily doses of 60 mL/day x 30 consecutive days. A 30 days follow-up without treatments was also established (day +60). ** P<0.05; *P< 0.01, and ***P<0.001 vs baseline and control.*

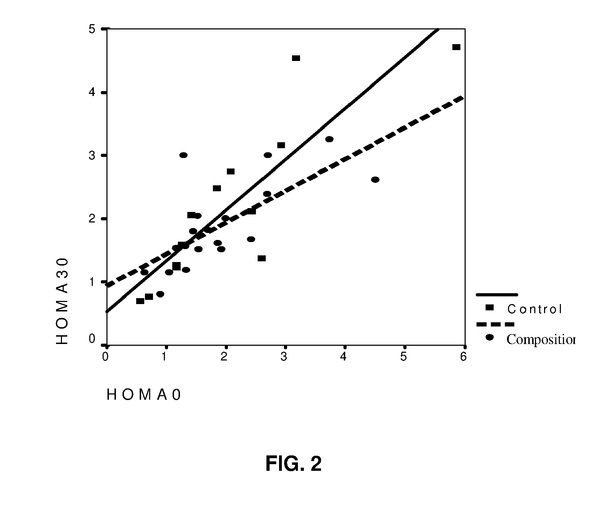

**Fig. 2****.** Evolution of the HOMA index of insulin resistance seen in patients with Chronic Kidney Disease. Comparison among the control group that received a conventional olive oil and the group that received the Composition, both at the same doses and schedule described in Fig. 1. *n.s: not significant due to the small size of the tail.*

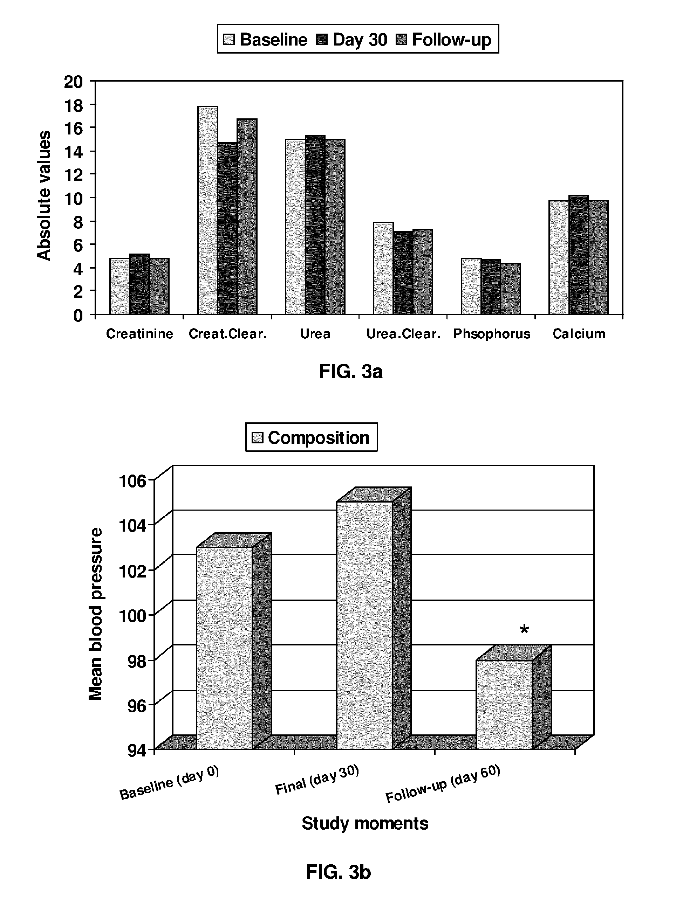

**Fig. 3****.** Effects of the Composition on (a) the evolution of parameters of kidney tolerance, and (b) mean blood pressure in patients with Chronic Kidney Disease. *Results in Fig. a are not significant (see the text);*P<0.05 vs baseline and day 30.*

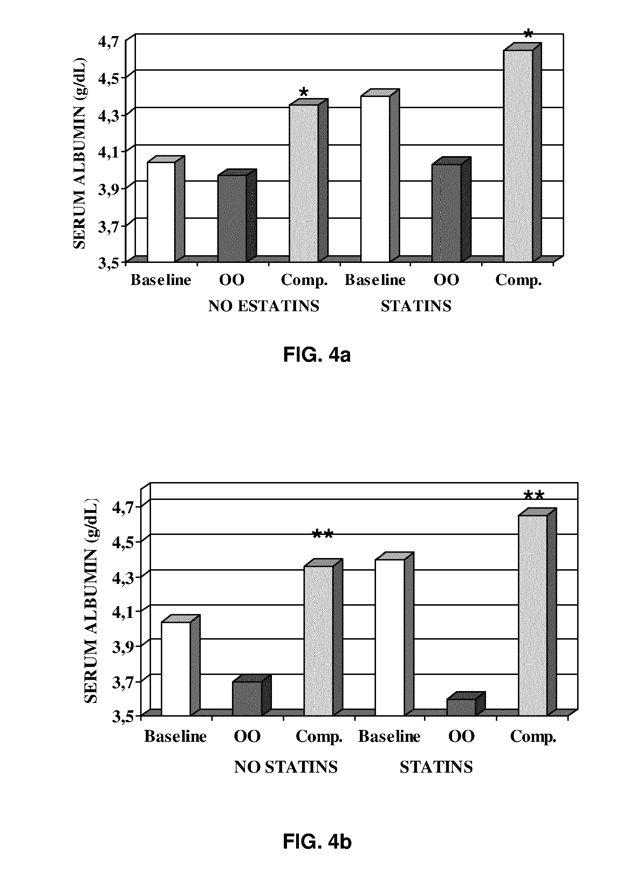

**Fig. 4****.** Effects of previous treatments wit statins on albumin serum levels in patients with Chronic Kidney disease: (a) in the whole groups receiving both treatments (b) only in responders to the conventional olive oil (OO) or the Composition (Comp.). * *P<0.05 y ** P<0.01 vs baseline and control.*

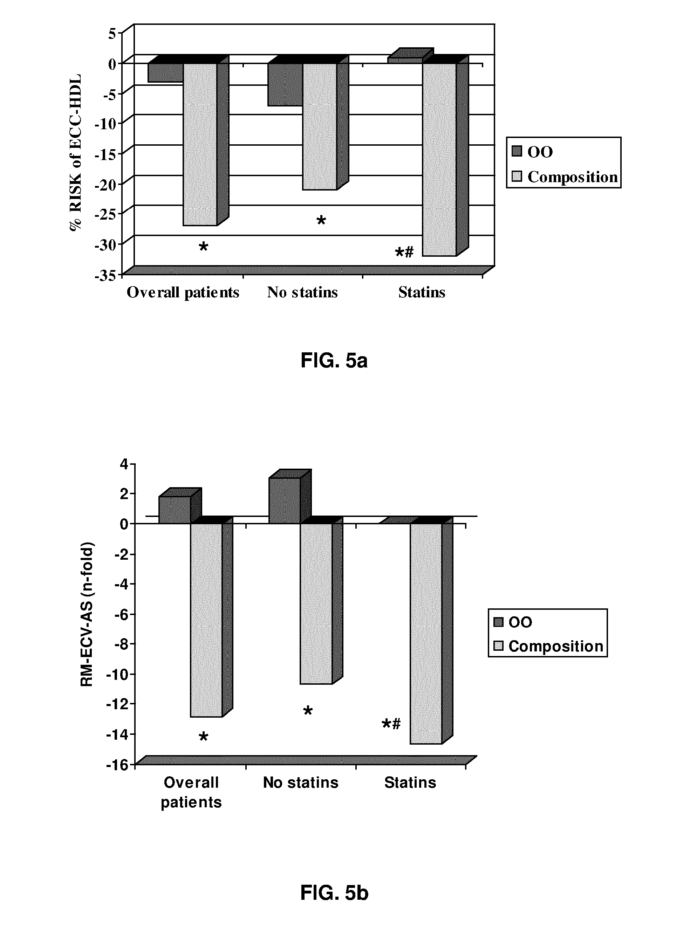

**Fig. 5****.** Effects of treatment with conventional olive oil (OO) or the Composition on the estimated cardiovascular risk (a) associated to HDL serum levels [R-ECC-HDL] (b) associated to albumin serum levels [RM-ECV-AS] in patients with Chronic Kidney Disease. * *Significant vs OO;* # *Significant vs Without Statins)*

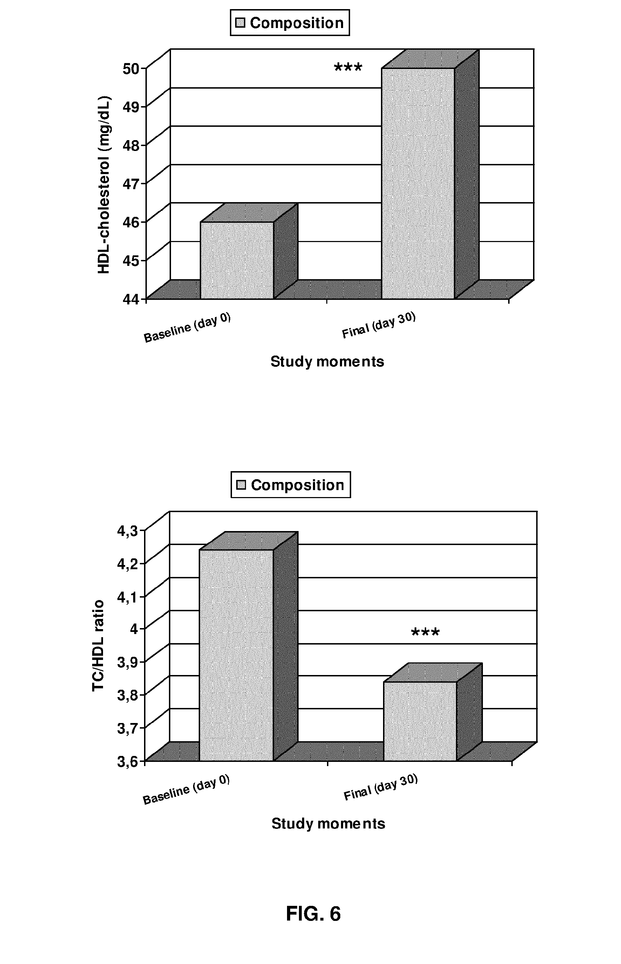

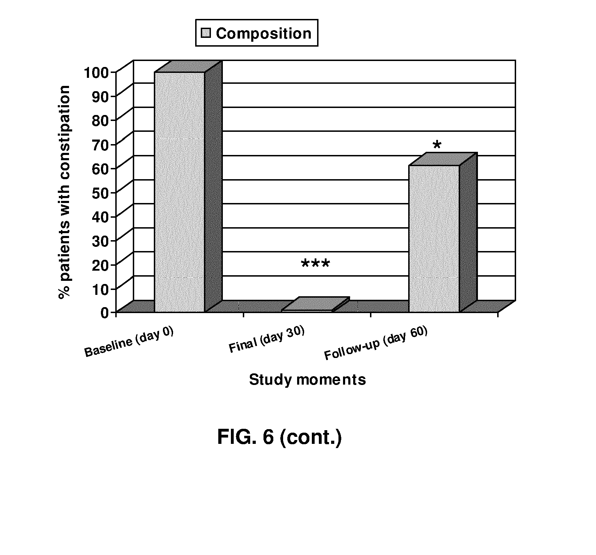

**Fig. 6****.** Effects of the Composition (60 mL/day x 30 days) on HDL-cholesterol serum levels, the total cholesterol(TC)/HDL-cholesterol ratio, and on constipation in a geriatric population. Before the entry to the study all persons were habitually olive oil consumers, and all were suffering from chronic constipation. * P<0.05, and *** *P< 0.001 vs baseline*

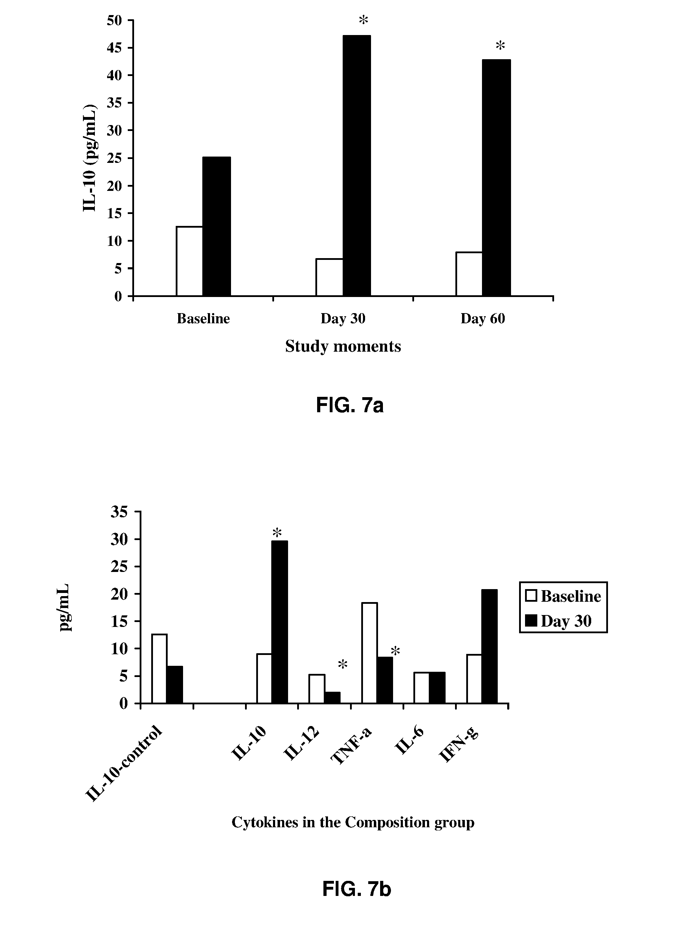

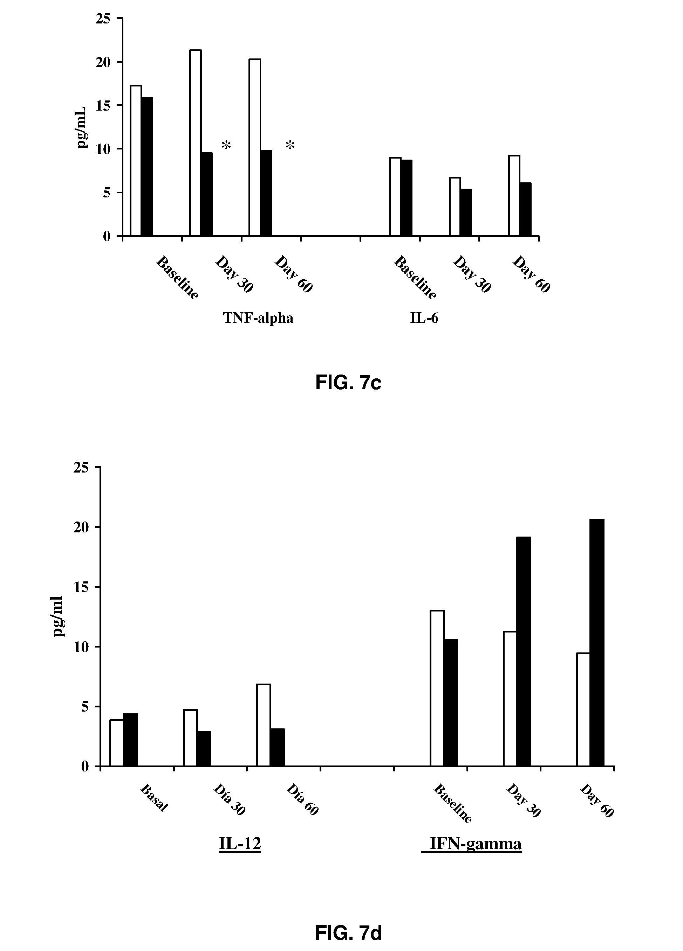

**Fig. 7**. Effects of the Composition (■) on evolutive serum levels of (a) interleukin-10: IL-10, (b) cytokines in low IL-10 producers, (c) tumour necrosis factor alpha: TNF-α and interleukin-6: IL-6, and (d) interleukin-12: IL-12 and interferon gamma: IFN-γ in patients with Chronic Kidney Disease. Comparison with the control group (□) that received a conventional olive oil. In both groups treatments were given by the oral route at doses of 60 mL/day x 30 consecutive days. A 30 days follow-up period without treatments was also established (day 60). **P<0.05 vs baseline and control*

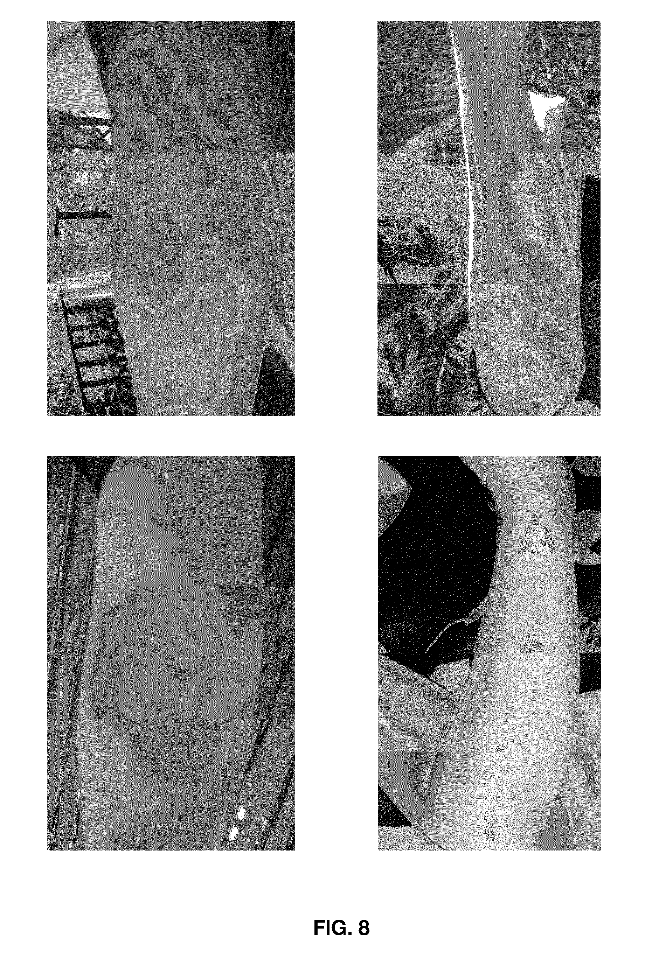

**Fig. 8****.** Effects of the oral intake of the Composition (60 mL/day x 45 consecutive days) on clinical evolution of a case of generalized, severe-recalcitrant atopic dermatitis: a) y b), before treatment; c) y d) after treatment

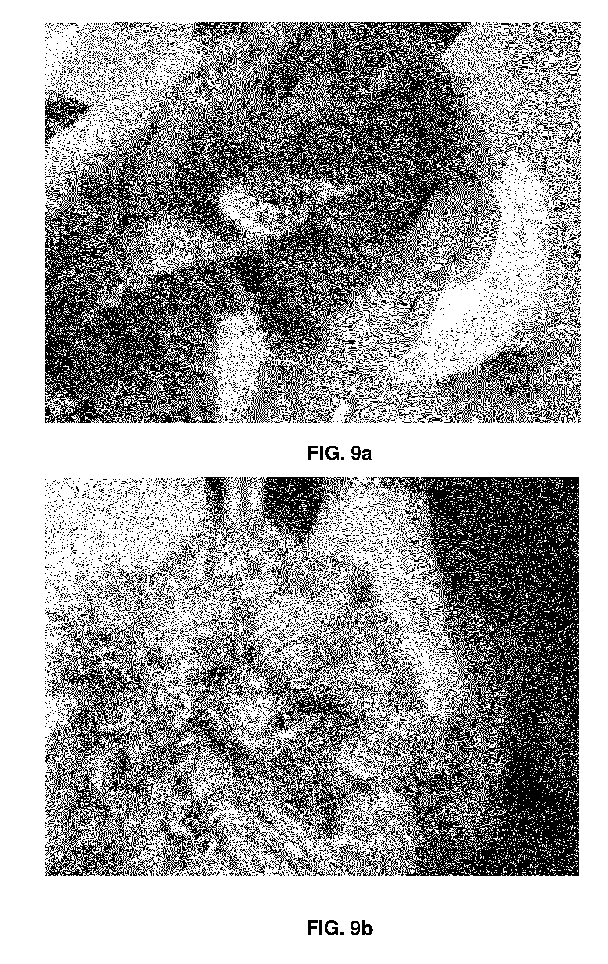

**Fig. 9****.** Evolution of a case of canine atopic dermatitis after the topic application of a gel containing the Composition plus urea. See the periorbicular affectation (a) before, and (b) after treatment.

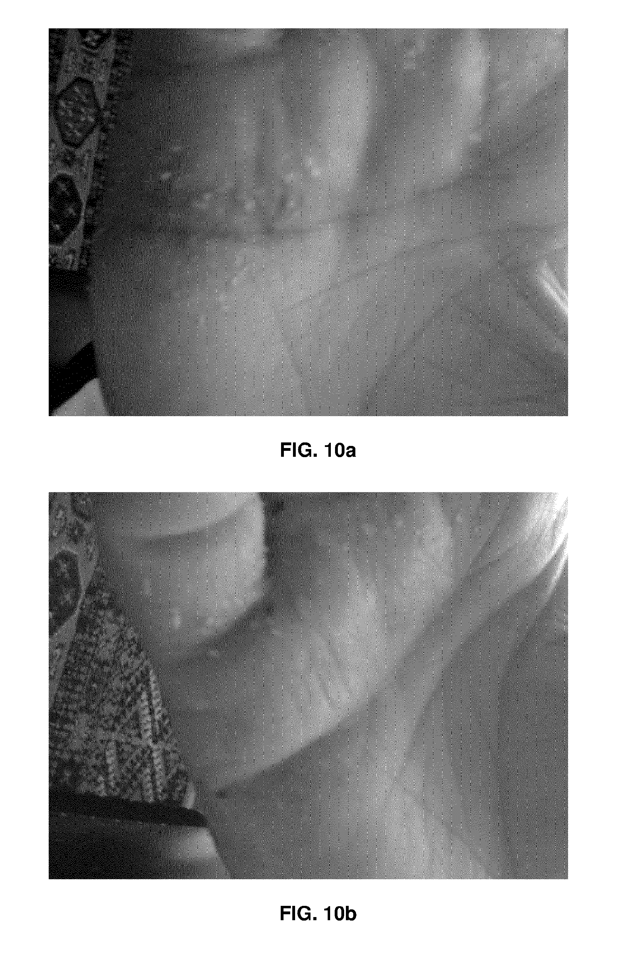

**Fig. 10****.** Evolution of a human case of atopic dermatitis with severe "hand eczema", resistant to conventional treatments, after the combined treatment with the Composition (orally given for 30 consecutive days), and the topic application of a cream (x 15 days) containing the Composition plus urea. (a) before, and (b) after treatments

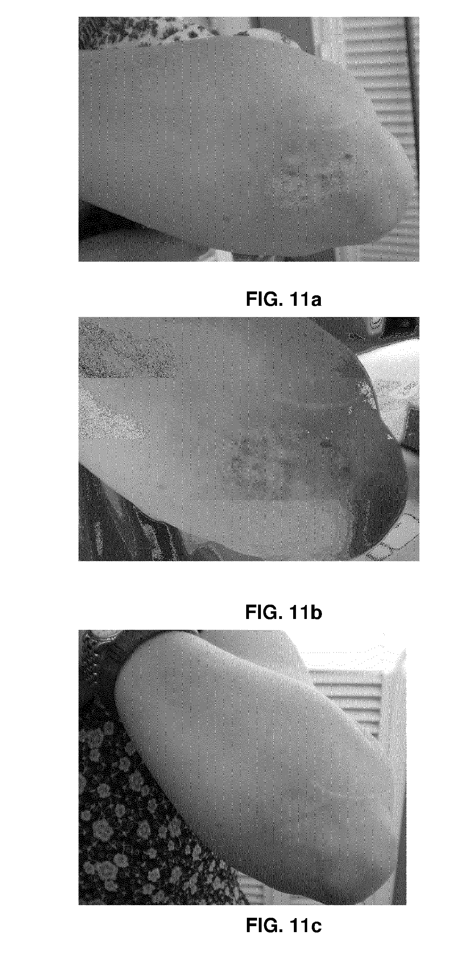

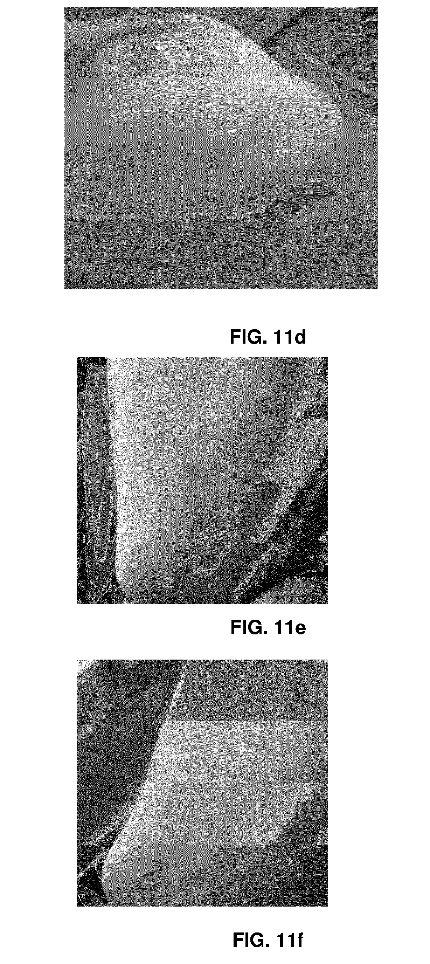

**Fig. 11****.** Effects of the oral intake (60 mL/day x 45 consecutive days) of the Composition, and the topic application of a gel containing the Composition*, on the evolution of a case of corticoid-resistant psoriasis: a) pre-treatment; b) 15 days after treatments; c) 45 days after treatments; and d) a follow-up of 45 days without treatments. Details on peripheral pigmentation (e) and hair growth (f) in the patient described in Fig. 11. *A 45 years old woman with facial acne, diagnosed of psoriasis 20 years ago; with familial history of atopic dermatitis and psoriasis. Treated with multiple cycles of topic corticosteroids, lesions relapsed before 15 days after the stop of corticosteroids. (* topic application of a gelified oil (the Composition) plus Rose Hip oil, aloe vera oil, and bisabolol.*

### EXAMPLES

Now, the Invention will be illustrated with 4 studies (carried out in 3 clinical trials) performed by the Inventors. These studies clearly show the specificity and clinical efficacy of the Composition.

### EXAMPLE 1

*Trial of nutritional intervention with the Composition in patients with Chronic Kidney Disease at pre-dialysis: Effects on albumin and HDL-cholesterol serum levels, and on Syndrome of Insulin resistance, blood pressure, and constipation. Safety and tolerance. Influence of statins withdrawal*

1. Ractionale and Procedures Patients with Chronic Kidney Disease (CKD) develop a picture of progressive malnutrition and inflammation, that is responsible for their elevated risk of cardiovascular disease. In an open, controlled-randomized study, the efficacy and safety of the Composition was compared with a conventional olive oil (OO) in 32 patients whose main clinical characteristics are shown in **Table 1**. After a 7 days withdrawal for ECA inhibitors and statins, 19 patients received the Composition (60 mL/day, t.i.d) for 30 consecutive days, and 13 remained as a control group with conventional olive oil with the same schedule of treatment. A 30 days follow-up period without treatments was established (day 60).

**Table 1. Demographic and clinical characteristics of the study populations. M. males; F: females; OO: conventional olive oil; CKD: chronic kidney disease; * According to KDIGO classification. No significant differences were detected between groups at baseline (before entry to the study).**

| | | **STUDY GROUPS** | |
|---|---|---|---|
| **PATIENT CHARACTERISTICS** | | **Control OO** | **Composition** |
| **OVERALL PATIENTS WITH CKD** (%) | | 13 (100) | 19 (100) |
| **Gender** (M/F) | | 7 / 6 | 9 / 10 |
| **Age, in years:** X±SD | | 61.4 ± 16.4 | 60.3 ± 10.1 |
| | • Women | 59.5 ± 20.5 | 60.9 ± 10 |
| **Habitually 00 Consumers** | | 13 (100) | 19 (100) |
| **Patients with constipation** | | 12 (92.3) | 17 (89.5) |
| **Previous treatment with statins** | | 6 (46.1) | 10 (52.6) |
| **Parameters of CKD:** | | | |
| | • Creatinine clearance (mL/min) | 18.9 ± 8.2 | 17.8 ± 7 |
| | • Urea clearance (mL/min) | 8.5 ± 3.4 | 7.9 ± 2 |
| | • Plasma creatinine (mg/dL) | 4.1 ± 2.1 | 4.8 ± 1,8 |
| | • Plasma urea (mg/dL) | 133 ± 51 | 150 ± 42 |
| **No. and (%) of patients at stage 5*** | | **4 (30.7)** | **7 (36.8)** |

### 2. Results

*2.a. Study drops:* the trial was initially defined for 2 groups of 20 patients each with Intention to Treat. During the study only 1 patient in the Composition group dropped, and 7 in the OO group. Thus, the Finally Treated groups were of 19 in the Composition and 13 in the control with 00 (**Table 1**).

**Table 2** shows the results (mean ± standard deviation) concerning Nutrition and Metabolic parameters in both groups. Results represent the profiles related to protein and albumin serum levels, lipids, and glucose parameters at baseline (day 0) and at the end of the study (day 30). In that concerns Nutrition profile, the Composition provoked not significant increases in weight, Body Mass Index (BMI), and total serum proteins (p=0.056). These serum increases were significant for albumin (p<0.05). On the contrary, all these parameters decrease (not significant) in the control OO group (**Table 2**). On day 60 (follow up) all nutrition values in the Composition group returned to baseline ones (**Fig. 1****, albumin).**

**Table 2** also shows that triglycerides slightly (not significant) increased in both groups of treatment. Total cholesterol increased significantly in the Composition group (p<0.05), and not significantly in the control. LDL-cholesterol plasma levels did not show modifications in any group. Finally, HDL-cholesterol serum levels increased significantly (p<0.01) only in the group receiving the Composition. These changes in the Composition group were translated into significant (P<0.05) reductions in the total colesterol/HDL ratio (**Fig. 1**). During the follow-up (day 60), triglycerides and LDL levels increase not significantly (data not shown), while serum numbers of total cholesterol (data not shown) and HDL (**Fig. 1****, HDL**) diminished just to reach the values at baseline. Not significant changes in the glucose profile were detected for both groups **(Table 2**). At baseline 1 patient showed a pathologic HOMA index in the control group, that increased to 2 patients throughout the study. On the contrary, the 2 patients with pathologic HOMA index in the Composition group at baseline, became normalized at the end of the study (day 30). **Fig. 2** shows the evolutive HOMA tendence during the study in both groups of patients. At follow-up 1 of the patients that had became normalized in the Composition group returned to exhibit a pathologic HOMA index (**Table 2**).

**Table 2. Effects of intake of the Composition (Comp.) (60Ml/day x 30 consecutive days) on nutrition and metabolic parameters in patients with Chronic Kidney Disease. Comparison with a control group that received conventional olive oil (OO). n: number of cases evaluated; Not significant statistical differences among groups were detected at baseline; # P=0.056, * P<0.05, and ** P<0.01 vs baseline and control group. A pathologic HOMA index is defined herein as of ≥ 3.9 in women, and of ≥ 3.5 in men. Values between bars represent the number of patients with pathologic HOMA index, and mean blood pressure values during the follow-up (day +60) in the Composition group.**

| | **TIME OF EVALUATION/GROUPS** | | | |
|---|---|---|---|---|
| **NUTRITION & METABOLIC PARAMETERS** | **BASELINE (Day 0)** | | **END (Day +30)** | |
| | **Control** **n=13** | **Comp.** **n =19** | **Control** **n=13** | **Comp.** **n=19** |
| **NUTRITION PROFILE** | | | | |
| Weight (Kgs) | 66.6 ± 12.9 | 73.6 ± 10 | 66.5 ± 13 | 74 ± 10.8 |
| Body Mass Index (Kg/m² | 27.9 ± 3.2 | 28.4 ± 4.6 | 27.8 ± 3.2 | 28.6 ±4.8 |
| Total proteins (g/dL) | 6.95 ± 0.5 | 6.9 ± 1.3 | 6.9 ± 0.5 | **7.4 ± 0.5^{#}** |
| Albúmina (g/dL) | 4.1 ± 0.3 | 4.1 ± 0.8 | 4.0 ± 0.3 | **4.5 ± 0.4*** |
| **LIPID PROFILE** | | | | |
| Triglycerides (mg/dL) | 112 ± 70.2 | 129 ± 62.7 | 115 ± 65.1 | 132 ± 51,4 |
| Total cholesterol (mg/dL) | 187 ± 44.2 | 192 ± 59.5 | 188 ± 59.5 | 214 ± 51.1* |
| LDL-cholesterol (mg/dL) | 115 ± 28.9 | 114 ± 43.9 | 115 ± 38 | 124 ± 40 |
| HDL-cholesterol (mg/dL) | 49 ±11 | 53 ±16.3 | 50 ± 11.5 | **63 ± 20.9**** |
| Total cholesterol/HDL ratio | 3.83 ±0.60 | 3.72 ± 0.87 | 3.74 ± 0.68 | 3.56 ± 0.91 |
| **GLUCOSE PROFILE** | | | | |
| Glucose (mg/dL) | 89.2 ± 9.6 | 91 ±14.7 | 88.5 ± 11.7 | 94.3 ± 8.6 |
| Insulin (µU/mL) | 9.4 ± 6.2 | 8.2 ± 3.4 | 10.5 ±5.8 | 8 ±2.7 |
| HOMa index | 2.1 ± 1.4 | 1.9 ± 0.97 | 2.2 ± 1.3 | 1.9 ± 0.69 |
| | | | | |
| **No and (%) of cases with pathologic HOMA** | 1 (7.7) | 2 (10.5) | 2 (15.4) | 0 (0) [1 (5)] |
| **Mean blood pressure** | 104 | 103 | 104 [103] | 105 **[98]**** |

### 2.b. Therapeutic Efficacy

Therapeutic efficacy was evaluated through the intensity of the response, as well as through the number of patients showing changes in all parameters in both groups of treatment. As shown in **Table 3** the Composition provoked significant increases in serum albumin and HDL-cholesterol, and significant decreases in total cholesterol/HDL ratio and HOMA index, when compared with the control group.

**Tabla 3. Evaluation of responses after the oral administration (60 mL/day x 30 days) of conventional olive oil (OO) or the Composition. Responders: patients showing increases of serum albumin and HDL cholesterol, and decreases in the total cholesterol/HDL ratio and HOMA index throughout the study; High Responders: patients showing decreases in LDL cholesterol serum levels while increasing HDL at levels of >7.5% with respect to baseline (these quantitative changes are thought to be associated to the diminution of the atherosclerotic plaque in patients treated with statins: Nicholls SJ, et al. Statins, high-density lipoprotein cholesterol, and regression of coronary atherosclerosis. JAMA 2007; 297:499-508); n: number of patients evaluated; n.s: not significant.**

| | **No of patients and (%)** | | |
|---|---|---|---|
| **RESPONSES** | **OO**, n: 13 | **Composition,** n:19 | **P value** |
| **SERUM ALBUMIN** (g/dL) | | | |
| Mean variation (range) | -0.06 (-0.4 to 0.1) | 0.42 (0 to 2.3) | < 0.05 |
| **No. and (%) of Responders** | **2 (15)** | **14 (74)** | < 0.001 |
| **HDL CHOLESTEROL** (mg/dL) | | | |
| Mean variation (range) | 1.23 (-8 to 8) | 10.73 (-7 to 51) | <0.01 |
| **No. and (%) of Responders** | **9 (69)** | **13 (68)** | n.s |
| Mean variation in Responders (range) | 3.55 (1 to 8) | 17.23 (5 to 51) | <0.01 |
| **No. and (%) of High Responders** | 4 (31) | 13 (68) | < 0.05 |
| Mean variation (range) | 5.5 (4 to 8) | 17.23 (5 to 51) | <0.01 |
| **TOTAL CHOLESTEROL/HDL RATIO** | | | |
| Mean variation (range) | -0.09 (-1.57 to 0.78) | -0.16 (-1.10 to 1.76) | <0.05 |
| **No. and (%) of Responders** | **7 (54)** | **13 (68)** | n.s |
| **HOMA index*** | | | |
| Mean variation (range) | 0.12 (-1.22 to 1.37) | -0.05 (-1.87 to 1.72) | n.s |
| **No. and (%) of Responders** | **3 (23)** | **10 (53)** | n.s |

**Table 4** summarizes the results concerning intrinsic parameters of CKD at baseline (day 0) and at the end of both treatments (day 30). In the Composition group significant decreases in urea and creatinine clearances were detected (p<0,05), although without clinical symptomatology nor the need to change the therapeutic established schedule of treatment. Interestingly, not significant decreases were seen for phosphorus and bicarbonate blood levels. At follow-up (day 60) all parameters returned to baseline (**Fig. 3a**).

**Table 4: Effects of conventional olive oil (control) and the Composition on intrinsic parameters of chronic kidney disease. n: number of cases evaluated. * P<0.05 vs baseline.**

| | **TIME OF EVALUATION / GROUPS** | | | |
|---|---|---|---|---|
| **PARAMETERS** | **BASELINE (day 0)** | | **END (Day +30)** | |
| | **Control** n=13 | **Comp.** n=19 | **Control** n=13 | **Comp.** n=19 |
| **Hemoglobin** (g/dL) | 12.3 ± 1.5 | 12.2 ± 0.8 | 12.3 ± 1.7 | 12.3 ± 0.8 |
| **Calcium** (mg/dL) | 9.3 ± 0.6 | 9.7 ± 1.4 | 9.3 ± 0.6 | 10.2 ± 0.6 |
| **Phosphorus** (mg/dL) | 4.2 ± 1.3 | 4.8 ± 1.3 | 4.2 ± 1.1 | 4.7 ± 1.2 |
| **Bicarbonate** (mEq/L) | 19.8 ± 3.7 | 21.4 ± 2.2 | 20.4 ± 3.8 | 20.9 ± 1.7 |
| **Urea** (mg/dL) | 133 ± 51 | 150 ± 42 | 138 ± 65 | 153 ± 39 |
| **Urea Clearance (mL/minute)** | 8.5 ± 3.4 | 7.9 ± 2 | 8.06 ± 4.1 | 7.06 ± 2.5* |
| **Serum creatinine** (mg/dL) | 4.1 ± 2.1 | 4.8 ± 1.8 | 4.3 ± 2.2 | 5.1 ± 1.6 |
| **Creatinine clearance (mL/minute)** | 18.9 ± 8.2 | 17.8 ± 7 | 19.4 ± 10.3 | 14.7 ± 6* |

### 2.c. Parameters of tolerance and safety

**Table 5** shows the absence of changes related to mean blood pressure, GPT, and homocisteine between baseline and the end of the study (day 30) in both groups. GOT increased significantly (p<0.05) in the Composition group. No changes were detected for constipation in the control group of conventional OO. In the Composition group drastic significant decreases (P<0.001) in the number of patients with constipation were detected. Thus, the percentage of patients with constipation diminished from 89.5% at baseline to 5.2% at the end of study (day 30). At follow-up (day +60) all parameters returned to baseline in patients taking the Composition, including the percentage of persons with constipation (**Fig. 1**). Interestingly, Mean Blood Pressure (MBP) decreased significantly (p<0.01) (**Fig. 3b**). The drastic decrease of constipation **(Table 5 and** **Fig.** 1) repercuted greatfuly in the Quality of Life of patients with CKD. Tolerance was excellent.

**Table 5. Effects of the Composition on Mean Blood Pressure (MBP), and other parameters of tolerance in patients with chronic kidney disease. GOT y GPT: transaminases; Control: patients taking a conventional olive oil; Comp.: composition; * P<0.05 vs baseline; # P<0.001 vs baseline and control; NS: not dignificant**

| | **TIME OF EVALUATIONS / GROUPS** | | | | **P values** | |
|---|---|---|---|---|---|---|
| **PARAMETERS** | **BASELINE (day 0)** | | **END (day +30)** | | **C0 *vs* C30** | **Comp0 *vs* Comp3** |
| | **Control** n=13 | **Comp** n=19 | **Control** n=13 | **Comp.** n=19 | | |
| **MBP** (mmHg) | 110 ± 13 | 103 ± 12 | 107 ± 11 | 105 ± 10 | NS | NS |
| **GOT** (UI/L) | 21 ± 10.3 | 16.5 ± 5.2 | 17.7 ± 6.9 | 19.4 ± 4.7* | NS | < 0.05 |
| **GPT** (UI/L) | 14.8 ± 8.4 | 15.5 ± 9.6 | 12.8 ± 4.9 | 16.6 ± 6.7 | NS | NS |
| **Homocysteine** (µg/L) | 24.6 ± 10.2 | 22.5 ± 6.4 | 27.4 ± 10.8 | 23.9 ± 8 | NS | NS |
| **No. and (%) of patients with constipation** | 12 (92.3) | 17 (89.5) | 12(92.3) | 1 (5.2)# | NS | < 0.001 |
| **No. and (%) patients that tolerate well the Composition** | - | - | - | 19 (100) | - | - |

### 2.d. Effects of statins withdrawal

Cause some patients in both groups had been taking statins before the entry to the study (**Table 1**), and because the long-lasting effects of statins on lipid metabolism are unknown, we evaluated the possible influence of statins in our study. As shown in **Table 6**, only the Composition showed slight additive effects on cholesterol HDL levels in patients that took statins. Furthermore, the Composition was able to increase HDL cholesterol by itself in the group that never had taken statins. Thus, it is clear the increases of HDL-cholesterol are due to the direct effects of the Composition. A possible synergistic/additive effect of the Composition with statins is suggested.

**Table 6. Effects of the Composition or a conventional olive oil (control) on HDL-cholesterol serum levels in CKD patients with or without previous treatment with statins. Responders: patients who showed HDL-c increases at day 30 (end of the study). In patients that had been taken statins, statins were withdrawal 1 week before the entry to the study; no differences in the number of patients taking statins were detected among both groups of treatment at baseline (Table 1). Increases or decreases (in negative numbers) are expressed in mg/dL/month; * P<0.05, ** P<0.01 y + P<0.005 vs control; # P=0.008 vs with statins.**

| **HDL-CHOLESTEROL INCREASES BETWEEN BASELINE (day 0) AND THE END OF TREATMENTS (day 30)** | **Control** n=13 | **Composition** n=19 |
|---|---|---|
| **INCREASES IN TOTAL RESPONDERS** (X±SD) | 51.8±11.7 | 70.6±19.9** |
| Mean increase in the total group | 1.23 | 10.73 * |
| • No. and (%) of responders | 9/13 (69) | 13/19 (68) |
| • Mean increase in responders | 3.55 | 17.23 + |
| **INCREASES IN RESPONDERS WITHOUT STATINS** | 52.8±13.8 | 67.8±17.7* |
| (X±SD) | 2.71 | 8.33 |
| Mean increase | 5/13 (38) | 5/19 (26) |
| •No. and (%) of responders | 4.80 | 18.40 # |
| • Mean increase in responders | | |
| **INCREASES IN RESPONDERS WITH STATINS** (X±SD) | 50.7±8.2 | 72.3±21.0+ |
| Mean increase | -0.5 | 12.90** |
| • No. and (%) of responders | 4/13 (31) | 8/19 (42) |
| • Mean increase in responders | 2 | 16.50+ |

### 2.e. HDL-cholesterol, serum albumin, and cardiovascular risks. Influence of the Composition and the statins withdrawal

Based on HDL-cholesterol and albumin serum levels achieved after both treatments, we evaluated the estimated risks of (a) coronary heart disease related to levels of HDL cholesterol (R-ECC-HDL) (b) cardiovascular mortality related to albumin serum levels in CKD patients (RM-ECV-AS). In the first case it is generally believed that each 1 mg/dL increase of plasma HDL cholesterol is associated, in the to a 2-3% decrease for the risk of coronary heart disease in the general population (Gordon DJ, Rifkind BM. High-density lipoprotein. The clinical implications of recent studies. N Engl J Med 1989; 321:1311-16*).* In the second case, it is accepted that for each 0.1 g/dL decrement in albumin level per month, the risk for cardiovascular death is 2.24- 3.86-fold greater among malnourished chronic kidney disease patients (Fung F, et al. Increased risk for cardiovascular mortality among malnourished end-stage renal disease patients. Am J Kidney Dis 2002; 40:307-14*).*

As shown in **Fig. 5a**, the increases of HDL-cholesterol provoked by the Composition (**Table 6**) were translated into a lower cardiovascular risk than that observed with the conventional olive oil (control). In the case of the Composition, these features are clearly independent of the previous treatment with statins, although a potentiation between the Composition and statins is suggested.

In that concerns serum albumin and mortality risk due to cardiovascular disease in CKD patients (**Fig. 5b**), the administration of olive oil (control) increased 2-fold this risk, while the administration of the Composition provoked significant decreases of 12.5-fold. In the case of control patients this risk can reach 3-fold in 69% of cases, while decreases of risk in the Composition group can reach 17.5-fold in 74% of cases (responders in **Table 3**).

Thus, the presence of a previous treatment with statins has little influence on the effects of the Composition on HDL cholesterol and albumin serum levels, and their associated cardiovascular risks. However, a synergistic effect of statins and the Composition is suggested.

### 2.f. Conclusions

The oral administration of the Composition, for a short period of time (30 days), to patients with chronic kidney disease: 1. Increases significantly albumin and HDL cholesterol serum levels; 2. Decreases the incidence of insulin resistance; 3. Decreases constipation drastically; 4. It has long-term effects on blood pressure reduction; 5. It appears to show synergistic/additive actions with statins. These effects are translated into a better nutritional and anti-inflammatory status that allow to a better quality of life. Acceptation of the Composition and tolerance were excellent.

### EXAMPLE 2

### Effects of the Composition on total cholesterol and HDL cholesterol serum levels, and on constipation, in a geriatric population

### 1. Rationale and procedure

Advanced ages are characterized by a high risk of cardiovascular disease as well as by deficiencies in the physical and mental performance status, both being related (among others) to low levels of HDL cholesterol. Constipation due to malnutrition and sedentarism affects negatively their quality of life, overall in females.

This is a pilot, open study, with one arm of nutrition intervention in 13 patients (11 women), with a mean age of 74.09±6.33 years old. Patients were required to meet 3 absolute inclusion criteria: (a) all were habitually consumers of conventional olive oil (OO) (b) all had chronic constipation for at least 6 months before the entry to the study; and, (c) persons that never took statins or other hypocholesterolemic products (fibrate, etc).

### 2. Results

2.a. None of persons dropped the study. Results at **Fig. 4** show that the Composition increased significantly (P<0.001) HDL cholesterol serum levels, while it provoked significant decreases in the total cholesterol/HDL ratio (P<0.001), and diminutions of constipation (P<0.001). Total cholesterol decreased also significantly **(Table 7**), and no changes of albumin serum levels were detected (data not shown).

| | **STUDY MOMENTS** | |
|---|---|---|
| **PARAMETERS** | **Baseline (n=13)** | **Final (day +30) (n=13)** |
| **TOTAL CHOLESTEROL** (mg/dL) | 196.25 ± 45.01 | 173.53 ± 49.63 * |
| Variation and (range) | | -20.53 (-57 to 2) |
| **No. and (%) of Responders** | | **12 (92)** |
| Mean variation (range) | | -22.41 (1 to 57) |

**Tab)e 7. Effects of the Composition (60 mL/day x 30 consecutive days) on total cholesterol and HDL cholesterol serum levels in elder people. Absolute values, and percentages of responders to treatment. TC/HDL: total cholesterol/HDL cholesterol ratio; n: number of persons evaluated; Responders: patients who showed decreases for total cholesterol and total cholesterol/HDL ratio, or increases in HDL cholesterol; High Responders: patients that showed decreases in LDL cholesterol together wit HDL cholesterol increases of > 7,5% between baseline and the end of treatment (see Table 3 for an explanation); * P<0.001 vs baseline**

| | | |
|---|---|---|
| **No. and (%) of Non Responders** | | **1 (8)** |
| Mean variation (range) | | 2 (2) |
| **HDL-CHOLESTEROL** (mg/dL) | 46.18 ± 10.41 | 49.76 ± 10.24 * |
| Mean variation and (range) | | 2.46 (-4.10 to 6) |
| **No. and (%) of Responders** | | **12 (92)** |
| Mean variation (range) | | 2.91 (1 to 6) |
| **No. and (%) og High Responders** | | **6 (46)** |
| Mean variation (range) | | 3.83 (3 to 6) |
| **TC/HDL RATIO** | 4.12 ± 0.73 | 3.47 ± 0.76* |
| **No. and (%) of Responders** | | **13 (100)** |
| Mean variation (range) | | -0.65 (-0.17 to -1.73) |
| **No. and (%) of High HDL Responders** | | **6 (46)** |
| Variation (range) | | -0.87 (-0.41 to -1.73) |

### 2.b. Therapeutic efficacy

As for CKD patients, the efficacy was evaluated through the intensity of the response, and the percentage of responders to the administration of the Composition. **Table 7** shows that responses were close to 100%, with near of 50% of high responders for HDL cholesterol and the CT/HDL ratio (according to Nicholls SJ, et al. Statins, high-density lipoprotein cholesterol, and regression of coronary atherosclerosis. JAMA 2007; 297:499-508*).* Constipation disappeared in almost 100% of cases (**Fig. 6**).

### 2.c. Conclusions

The intake of the Composition for a short period of time (30 days) in elder people: 1. Increases HDL cholesterol serum levels; 2. Decreases total cholesterol and the TC/HDL ratio; 3º. Reducess constipation drastically. These results (a) potentiate those data reported for CKD patients (b) are translated into a better nutrition status, together with a potent antiinflamatory and cardiovascular protector situation. Acceptation and tolerance were excellents.

### EXAMPLE 3

### Effects of the Composition on immune/inflammatory parameters in patients with chronic kidney disease (CKD) at pre-dialysis. Changes in inflammatory (IL-6, TNF-α) and regulatory (IL-10, IL-12 e IFN-γ) cytokines. Comparison with patients receiving conventional olive oil

### 1. Rationale and Procedures

Patients with CKD exhibit a proinflammatory picture characterized by decreases in interleukin-10 (IL-10) production, and the derailment of proinflammatory cytokines such as interleukin-6 (IL-6) and tumor necrosis factor alpha (TNF-α). These alterations increase with advanced ages. In the trial described as EXAMPLE 1, sera from patients were collected at baseline (before entry to the study), day 30 (end of treatments), and at follow-up without treatments (day 60). Interleukins 6, 10, 12 (IL-6, IL-10 and IL-12), as well as TNF-α and interferon gamma (IFN-γ) were determined by ELISA. All determinations were done by duplicate and tested again by an independent laboratory.

### 2. Results

Taking into account the extreme inter-patient variations in cytokine blood levels, we only describe herein the most notable features seen (**Fig 7**). In that concerns IL-10 (**Fig. 7a**), we only detected significant increases along the study in the Composition group. Cause at baseline IL-10 levels were higher (but not significant) in the Composition group, we decided to evaluate only those patients showing similar low IL-10 values (low producers) in both groups of treatment at baseline. Results definitively showed that only the Composition was able to increase IL-10 serum levels in CKD (**Fig. 7b**).

With regards to TNF-α (**Fig. 7c**), we detected significant decreases throughout the study, only in the Composition group. Figs. **7c** and **7d** show the statistical tendencies for IL-6, IL-12, and IFN-γ.

### 3. Therapeutic Efficacy

**Table 8** shows the number and percentage of CKD patients who exhibited (a) increases in IL-10 and IFN-γ serum levels (b) decreases of IL-6, TNF-α and IL-12 serum levels at day 30 (end of treatments).

**Table 8. Number and percentage of CKD patients who showed changes in serum cytokine levels after the administration of a conventional olive oil (OO) or the Composition (60 mL/day x30 consecutive days). *P<0.05; **P<0.01 y # P=0.058 vs control**

| | **STUDY GROUPS** | |
|---|---|---|
| **CYTOKINE CHANGES** | **Control (OO)** | **Composition** |
| **Increases**: | | |
| IL-10 | 4/11 **(36.36)** | 15/19 **(78.94)*** |
| IFN-γ | 2/11 **(18.18)** | 16/19 **(84.21)**** |
| **Decreases:** | | |
| IL-6 | 3/11 **(27.27)** | 15/19 **(78.94)**** |
| TNF-α | 5/11 **(45.45)** | 15/19 **(78.94)^{#}** |
| IL-12 | 2/11 **(18.18)** | 15/19 **(78.94)**** |

### 4. Conclusions

These results show by the first time the immunomodulatory activity of the Composition in patients with chronic kidney disease. In fact, the proinflammatory response was decreased while the immune mechanisms involved in the host resistance were preserved. These systemic features contribute also to explain the activity of the Composition on other inflammatory/oxidative diseases, as they are the cases of atopic dermatitis and psoriasis (see below). In fact, T regulatory cells (Tregs) producing IL-10 and IFN-γ are diminished in all these systemic and skin pathologies.

### EXAMPLE 4

### Effects of the combined treatment (oral and topic) with the Composition in patients and dogs with severe atopic dermatitis and in psoriasis patients.

### 1. Rationales and Procedures

Atopic dermatitis (AD), with or without hand eczema, and Psoriasis represent serious and growing problems of Public Health. We treated with the Composition 8 cases de AD, all diagnosed by their Dermatologists, whose main clinical characteristics are summarized in **Table 9**. All of them were habitually consumers of conventional or organic olive oils. Case No. 7 suffered also of recurrent plaque psoriasis since more than 20 years. Case No. 8 had bilateral severe Hand Eczema. Previous treatments were the habitual in these cases (hydratation, emollients, oral and topic corticosteroids, etc), and cases 6, 7 and 8 had also received several courses with topic inhibitors of calcineurin (TIC). Corticosteroids and/or TIC were withdrawal 3 months before the entry to this study.

**Table 9. Main clinical characteristics of patients included to be treated orally and/or topically with the Composition. F: female; M: male; * Brothers; # Father and daughter; CH: clinical history.**

| **Case s** | **Age in years (gender)** | **Time from diagnoses** | **Intensity of the illness** | **Other signs related to atopy** |
|---|---|---|---|---|
| **1** | 6 (F)* | 1 year old | Mild to moderate | Active asthma |
| **2** | 7 (M)* | 1 year old | Mild to moderate | Active asthma |
| **3** | 11 (M)* | 1 year old | Mild to moderate | Asthma in |
| **4** | 18 (F)# | 1 year old | Mild to moderate | remission |
| **5** | 55 (M)# | Infancy | Mild to moderate | CH Allergic rhinitis |
| **6** | 36 (F) | 20 years old | Recalcitrant, severe | No |
| **7** | 45 (F) | Infancy | Recalcitrant | CH Allergic rhinitis |
| **8** | 70 (M) | Infancy | Recalcitrant | CH rhinitis and psoriasis Hand Eczema |

All patients received the Composition by the oral route at doses indicated in **Table 9**. No other treatments were permitted, excepting for patients 1 to 3, 7, and 8, that were also treated with different topic lotions elaborated with the Composition (see Claims for this patent).

Recently it has been reported that canine dermatitis is similar to human dermatitis. For this reason we also treated a dog (Spanish of Waters) with recurrent atopic dermatitis (**Fig. 9**) and severe periorbicular affectation. The dog was washed twice a week with a gel containing the Composition.

### 2. Results

The evaluation at day 60 after treatments showed the results in **Table 10.** Quality of Life definitively changed in all patients (better sleep in children, and a radid social adaptation of cases 6 and 8 (visit to public swimming pools, no need for globes, etc).

Interestingly, the Complete Response of the psoriatic plaque of case 7 was accompanied by perilesional hyperpigmentation and hair growth (**Figs. 11e** **y f**), thus suggesting the ability of the topic formulation to act on skin cell progenitors (see Patent Claims for vitiligo and alopecia).

### 3. Conclusions

All treatments with the Composition were well tolerated, and no signs or symptoms of skin allergy were seen. Actually, patient number 7 is free of illness after 45 days without topical treatment, but maintaining the oral treatment with the Composition (30 mL/day, t.i.d). The amelioration of the Quality of Life is a common feature in all patients.

**Table 10. Previous treatments, and effects of the Composition in patients with atopic dermatitis, hand eczema, and psoriasis. CR: complete remission, with dissapearance of cutaneous signs and symptoms; PR: partial response; Hidrat: hydratation lotions; OGC: oral glucocorticoids, and TGC (topical glucocorticoids); TIC: topic inhibitors of calcineurin.**

| | | **Composition: oral administration for 60 days 60 days** | |
|---|---|---|---|
| **Cases** | **Previous Tratments** | **Doses, mL/day** | **Cutaneous Response** |
| 1 | Emollients+Hidratation | 10 | CR |
| 2 | Emollients+Hidratation+ OGC and TGC | 15 | CR |
| 3 | Emollients+Hidratation | 15 | CR |
| 4 | Emollients +Hidratation | 30 | CR |
| 5 | Emollients + Hidratation | 50 | CR* |
| 6 | Emollients, Hidratation, OGC y TGC, TIC | 50 | PR> 75% (Fig. 8) |
| 7 | Emollients, Hidratation, TGC, TIC | 50 | CR* (Figs. 11) |
| 8 | Emollients, Hidratation, TGC, TIC | 50 | CR /Fig. 10) |

### CONCLUSIONS TO EXAMPLES

In spite of the apparent diversity of biologic effects described herein, a unique common mechanism appears as the responsible for these effects: the ability of the Composition to regulate the endogenous production of IL-10 and IFN-γ. Moreover, today we know that different fatty acids, many of them contained in the Composition, act on nuclear receptors (PPARs ("peroxisome proliferator-activated receptors") localized at the small intestine, liver, and skin, thus regulating the immune/inflammatory response. These features will explain the activity of the Composition (*Villarrubia VG, et al. Epidermal barrier and lipid nutrition: personalyzing atopic dermatitis. I. Regulatory enzymes, and fatty acid-binding proteins (FABPs) engaged in the PPAR and immune connections. Villarrubia VG, et al. The epidermal barrier and lipid nutrition: personalyzing atopic dermatitis. II. The PPAR connection and inflammatory immunopathology as targets for new treatments) [manuscript in preparation*]

*It is of interest to note that during the elaboration of oil in water emulsions (O*/*W) for their topical use, the amounts of olive oil accepted oscilated from 3.5 to 7.5 % for the conventional picual and arbequino olive oil varieties, between 4,5 to 10% for the same organic olive oil varieties, and up to 87% for the Composition. These results, together with its low waxes content, highly contribute to explain the easy and quick absorption of the Composition when orally given or topically applied.*

## Claims

1. Composition that comprises at least:
■ Extra virgin olive oil of the Picual variety in a proportion of between 42% and 62% by volume
■ Extra virgin olive oil of the Arbequino variety in a proportion of between 20% and 40% by volume
■ Extra virgin olive oil of the Cornicabra variety in a proportion of between 8% and 28% by volume
■ Extra virgin olive oil of the Hojiblanca variety in a proportion of between 0% and 5% by volume
■ Extra virgin olive oil of the Empeltre variety in a proportion of between 0% and 5% by volume.

2. Composition, as claimed in claim 1, where the olive oil is obtained from ecological agriculture.

3. Composition, as claimed in any of claims 1 or 2, to be used as a drug.

4. Pharmaceutical preparation that comprises the composition, as claimed in any of claims 1 or 2, and a pharmaceutically acceptable adjuvant or carrier.

5. Pharmaceutical preparation, as claimed in claim 4, to be administered by oral or topical route.

6. Use of a composition, as claimed in any of claims 1 or 2, for the manufacturing of a drug designed for the treatment or prevention of a disorder selected from a set of disorders associated with serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders, inflammatory or oxidative disorders, or immunological disorders.

7. Use of the composition, as claimed in claim 6, where the disorder is selected from chronic kidney disease, diabetes and insulin resistance, hypercholesterolaemia, dyslipidaemia, hypertension, hypoalbuminaemia and hypoproteinaemia, kwashiorkor, cachexia, anorexia, bulimia, constipation, age-associated inflammatory processes, physical condition deficiencies, dermatitis and dermatosis.

8. Use of the composition, as claimed in any of claims 1 or 2, as a co-adjuvant with different therapies.

9. Use, as claimed in claim 8, where the therapy is selected from surgery, chemotherapy, radiotherapy, nutritional therapies, therapies with cholesterol-reducing agents, dialysis procedures and vascular stents, or administration of vaccines.

10. Use of the composition, as claimed in any of claims 1 or 2, as a carrier or vehicle of drugs, nutraceutic agents and/or nutritional therapies.

11. Use of the composition, as claimed in any of claims 1 or 2, for the treatment or prevention of a disorder selected from a set of disorders associated with serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders caused by vascular stents or by dialysis procedures, inflammatory or oxidative disorders, or immunological disorders.

12. Use of the composition, as claimed in any of claims 1 or 2, for the manufacturing of a nutraceutic agent or functional food.

13. Method designed for the treatment or prevention of disorders in a mammal, preferably a human being, which comprises the administration of a therapeutically effective quantity of a composition, as claimed in any of claims 1 or 2.

14. Treatment method, as claimed in claim 13, where the administration is by oral or topical route, or both simultaneously.

15. Treatment method, as claimed in any of claims 13 or 14, where the disorder is selected from a set of disorders associated with serum levels of cholesterol or protein-bound cholesterol, cardiovascular disorders, metabolic disorders, kidney disorders, neurological disorders, cancer, infections, disorders associated with intestinal absorption mechanisms, nutritional disorders and nutritional deficiencies, disorders of the dermis and skin appendages, degenerative disorders, iatrogenic disorders caused by vascular stents or by dialysis procedures, inflammatory or oxidative disorders, or immunological disorders.

16. Treatment method, as claimed in claim 15, where the disorder is selected from chronic kidney disease, diabetes and insulin resistance, hypercholesterolaemia, dyslipidaemia, hypertension, hypoalbuminaemia and hypoproteinaemia, kwashiorkor, cachexia, anorexia, bulimia, constipation, age-associated inflammatory processes, physical condition deficiencies, dermatitis and dermatosis.

17. Dermocosmetic or dermatological preparation that comprises at least between 0.05% and 95% of the composition, as claimed in any of claims 1 or 2.

18. Dermocosmetic or dermatological preparation that comprises at least between 5% and 90% of the composition, as claimed in any of claims 1 or 2.

19. Dermocosmetic or dermatological preparation that comprises at least between 10% and 80% of the composition, as claimed in any of claims 1 or 2.

20. Dermocosmetic or dermatological preparation, as claimed in any of claims 17 to 19, which additionally comprises other ingredients, selected from urea, shea butter, vitamin E, rose hip, aloe vera, bisabolol, lanolin or olive pit, seed and/or pulp extract or olive tree leaf extract.

21. Dermocosmetic or dermatological preparation, as claimed in claim 20, where the other ingredient is in a proportion of between 0.05% and 95% by volume.

22. Dermocosmetic or dermatological preparation, as claimed in claim 21, where the other ingredient is in a proportion of between 5% and 90% by volume.

23. Dermocosmetic or dermatological preparation, as claimed in claim 22, where the other ingredient is in a proportion of between 10% and 80% by volume.

24. Method designed for the treatment or prevention of a skin disorder in a mammal that comprises topically applying a therapeutically effective quantity of the dermocosmetic or dermatological composition, as claimed in any of claims 17 to 23, on the area to be treated.

25. Treatment method, as claimed in claim 24, where the skin disorder is selected from atopic dermatitis, hand eczema, psoriasis, hyperkeratosis, pemphigus, vitiligo and other pigmentary disorders, processes that affect the skin and nails, bullous epidermolysis, burns caused by external agents, intrinsic and extrinsic skin ageing, iatrogenic disorders caused by vascular stents or by treatments with corticoids, cytostatic agents, retinoic acid and the derivatives thereof, psoralens associated with ultraviolet radiation or other natural and/or synthetic substances for topical use, tumours or natural or iatrogenic infections of the skin and skin appendages.

26. Use of a dermocosmetic or dermatological preparation, as claimed in any of claims 17 to 23, for the treatment or prevention of a skin disease.

27. Use of a dermocosmetic or dermatological preparation, as claimed in any of claims 17 to 23, as a co-adjuvant in a therapy.

28. Use, as claimed in claim 27, where the therapy is selected from light therapies with laser or ultraviolet radiations, photochemotherapy, surgery, chemotherapy, radiotherapy, nutritional therapies, implantation of vascular stents or therapies with corticoids or retinoic acid and the derivatives thereof.
